Europäisches Patentamt

European Patent Office

Office européen des brevets

(11) Veröffentlichungsnummer: **0 353 375 A1**

# (12) EUROPÄISCHE PATENTANMELDUNG

(21) Anmeldenummer: 88890202.0

(22) Anmeldetag: 02.08.88

(51) Int. Cl.⁴: **A61C 13/23**

(43) Veröffentlichungstag der Anmeldung:
07.02.90 Patentblatt 90/06

(84) Benannte Vertragsstaaten:
**AT BE CH DE ES FR GB GR IT LI LU NL SE**

(71) Anmelder: **Altwirth, Oskar**
**Oberach 37**
**A-4950 Altheim(AT)**

(72) Erfinder: **Altwirth, Oskar**
**Oberach 37**
**A-4950 Altheim(AT)**

(74) Vertreter: **Hübscher, Gerhard, Dipl.-Ing. et al**
**Patentanwälte Dipl.-Ing. Gerhard Hübscher**
**Dipl.-Ing. Helmut Hübscher Dipl.-Ing. Heiner**
**Hübscher Spittelwiese 7**
**A-4020 Linz(AT)**

(54) **Hafteinlage für Zahnprothesen und Verfahren zu deren Herstellung.**

(57) Eine Hafteinlage (1) für Zahnprothesen besteht aus einem haftstoffdurchsetzen Faservlies (2, 3).

Um eine gute und vor allem auch haltbare Haftwirkung zu erreichen, nimmt das Faservlies (2) einen Haftstoff (3) aus einer pastösen Mischung von Alginat und/oder Carboxymethylcellulose, Polyvinylacetat und einem alkoholischen Lösungsmittel auf.

EP 0 353 375 A1

## Hafteinlage für Zahnprothesen und Verfahren zu deren Herstellung

Die Erfinding bezieht sich auf eine Hafteinlage für Zahnprothesen, insbesondere Unterkiefer-Zahnprothesen, bestehend aus einem haftstoffdurchsetzen Faservlies, sowie auf ein Verfahren zum Herstellen solcher Hafteinlagen.

Um die Haftfähigkeit und Wirkungsdauer rein pastöser Haftmittel für Zahnprothesen zu erhöhen, gibt es gemäß der DE-C-24 13 380 bereits Hafteinlagen aus einem Faservlies, das mit Haftstoff durchsetzt ist, welches Faservlies beim einsatz der Hafteinlage zwischen Prothese und Kiefer durch seine Fasern gewissermaßen eine Armierung des Haftstoffes bildet und das Ausspülen des Haftstoffes verzögern soll. Allerdings ist bei den bekannten Hafteinlagen das Faservlies zusammen mit einem trockenen pulverförmigen Hydrokolloid als Haftstoff verpreßt, das durch ein Befeuchten aufquellt und aktiviert wird, so daß es eine gewisse Menge Flüssigkeit bis zur Sättigung aufnehmen und dadurch einen Adhäsionseffekt bewirken, doch auch durch Flüssigkeit und Speichel ausgespült werden kann. Die Wirkungsdauer dieser Hydrokolloide oder andere Quellmittel enthaltenden Hafteinlagen bleibt daher unbefriedigend; sie sind nicht in der Lage, insbesondere Unterkiefer-Zahnprothesen wegen ihrer recht geringen Haftflächen mit gewünschter Sicherheit und Wirkungsdauer zu fixieren.

Der Erfindung liegt daher die Aufgabe zugrunde, diese Mängel zu beseitigen und eine Hafteinlage der eingangs geschilderten Art zu schaffen, die sich durch ihre besonders gute und lang anhaltende Haftwirkung auszeichnet und auch bei Unterkiefer-Zahnprothesen einen dauerhaften Festsitz gewährleistet. Außerdem soll ein Verfahren zur rationellen Herstellung solcher Hafteinlagen angegeben werden.

Die Erfindung löst diese Aufgabe dadurch, daß das Faservlies einen Haftstoff aus einer pastösen Mischung von Alginat und/oder Carboxymethylcellulose, Polvinylacetat und einem alkoholischen Lösungsmittel aufnimmt. Dieser Haftstoff, der in ähnlicher Form als Haftcreme bereits aus der DE-C 35 46 367 bekannt ist, erlaubt nicht nur die Nutzung seiner Vorzüge als Kleber, wie Abspülsicherheit durch die Einbettung des Alginates beziehungsweise der Carboxymethylcellulose in Polyvinylacetat und der durch das Polyvinylacetat selbst gegebene Klebeeffekt für die Hafteinlage, sondern bringt darüber hinaus noch einen besonders wirkungsvollen Unterdruck-Saufeffekt mit sich. Wird nähmlich die Einlage zwischen Prothese und Kiefer eingesetzt, entsteht durch Feuchtigkeitsaufnahme aus dem pastösen Haftstoff eine kompakte, gummiartige Masse, die durch das Faservlies eine strukturelle Festigung erfährt. Diese an den Haftflächen angepreßte Masse ergibt auf Grund der Druck-und Zugbelastung bei Kaubewegungen u. dgl. stark haftende Unterdruck- und Saugbereiche. Dieser Unterdruck-Saufeffekt garantiert zusammen mit dem Klebeeffekt der Einlage eine überraschend dauerhafte und fest haltende Haftverbindung zwischen Prothese und Kiefer und ermöglicht damit ein langzeitiges, sicheres Tragen auch von Unterkiefer-Zahnprothesen.

Um bei längerer Lagerung oder schlechter Verpackung ein Austrocknen der Hafteinlage zu verhindern, kann der Haftstoff einen physiologisch einwandfreien Weichmacher, vorzugsweise ein Propylen-Glykol oder ein Glycerin-Triacetat enthalten.

Ist erfindungsgemäß Haftstoff zwischen zwei Faservliesschichten eingebracht und auf die dem Kiefer zugeordnete Faservliesschicht außen aufgetragen, entsteht eine überaus wirkungsvolle Hafteinlage, die zuerst mit der freien Faservliesschicht auf die Prothese aufgedrückt wird, so daß der zwischen den Faservliesschichten sich befindende Haftstoff die prothesenseitige Faservliesschicht durchdringt und die Haftung zwischen Einlage und Prothese gewährleistet. Die Prothese läßt sich dann einfach mit der Haftstoffschicht voran auf den Kiefer aufsetzen, wodurch die gewünschte Haftwirkung auf für Unterkiefer-Zahnprothesen zustandekommt.

Ein sauberer, hygienischer Schutz ergibt sich, wenn auf die äußere Haftstoffschicht und vorzugsweise auch die äußere Faservliesschicht Deckfolien aufgelegt sind, welche beiden Deckfolien gegebenfalls unterschiedliche Farbe besitzen, um die ordnungsgemäße Anwendung der Hafteinlage zu erleichtern.

Gemäß einer vorteilhaften Weiterbildung der Erfindung wird zur rationellen Herstellung einer wirkungsvollen Hafteinlage eine Lösung aus feinzerteiltem Polyvinylacetat und vorzugsweise 85%-igem Äthylalkohol vorbereitet und in diese Lösung Alginat und/oder Carboxymethylcellulose eingebracht und die ganze Mischung bis zu einer pastösen Masse geknetet, mit welcher Masse dann Faservlies durchsetzt bzw. bestrichen wird. Dabei wird günstigerweise eine Lösung aus etwa 75% Polyvinylacetat und 43% Äthylalkohol aufbereitet und etwa 50 Gewichtsteile dieser Lösung werden mit 35 Gewichtsteilen Alginat oder Carboxymethylcellulose bzw. 7 Gewichtsteilen Alginat und 28 Gewichtsteilen Carboxymethylcellulose vermischt. Es entsteht ein Haftstoff, der die gewünschte Klebewirkung und zusammen mit dem Faservlies auch beste Saugeffekte mit sich bringt.

Werden zusätzlich zu 100 Gewichtsteilen der fertigen Polyvinylacetatlösung zumindest 50 Gewichtsteile des Weichmachers mittels Vakuum-Rührer zugemischt, läßt sich auf einfache und sichere Weise ein Austrocknen der Hafteinlage auch bei undichten Verpackungen oder langen Lagerzeiten verhindern, ohne dadurch die Haftklebeeigenschaften zu beeinträchtigen.

Besonders günstig ist es weiters, wenn erfindungsgemäß Haftstoff kontinuierlich auf eine Faservliesbahn aufgetragen, mit einer zweiten Faservliesbahn abgedeckt und leicht verpreßt wird, worauf die zweite Faservliesbahn mit Haftstoff bestrichen und das entstehende Band aus Faservlies- und Haftstoffschichten vorzugsweise beidseitig mit Deckfolien versehen wird, aus welchem Band anschließend der Prothesenform entsprechende Stanzlinge ausgestanzt und diese als gebrauchsbereite Haft einlagen stückweise luftdicht abgepackt werden. Es können Faservliesbahnen aus Zellulose mit einem Flächengewicht von ca. 45 g/m² bzw. 70 g/m² vorgesehen sein, auf deren erste Haftstoff in einer Menge von ca. 8 g/dm² und auf deren zweite Haftstoff in einer Menge von ca. 5 g/dm² aufgetragen wird. Auf wirtschaftliche Weise entstehen fertig abgepackte, jederzeit einsetzbare Hafteinlagen, die vor einem Austrocknen geschützt und daher lang lagerfähig sind.

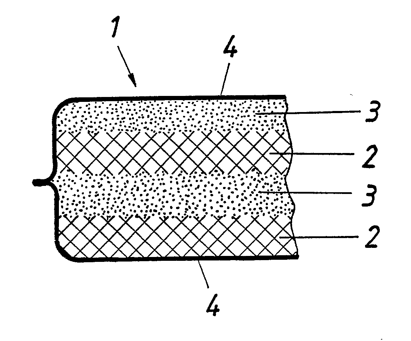

In der Zeichnung ist eine erfindungsgemäße Hafteinlage beispielsweise an Hand eines schematischen Querschnittes näher veranschaulicht

Um einen guten und dauerhaften Sitz vor allem einer Unterkiefer-Zahnprothese zu erreichen, ist eine Hafteinlage 1 vorgesehen, die eine dem Unterkiefer angepaßte U-förmige Gestalt aufweist und schichtenweise aus Faservlies 2 und Haftstoff 3 aufgebaut ist. Der Haftstoff 3 besteht dabei aus einer pastösen Mischung von Alginat und/oder Carboxymethylcellulose, Polyvinylacetat und einem alkoholischen Lösungsmittel und wird sowohl zwischen die beiden Faservliesschichten 2 als auch außen auf die kieferseitige Faservliesschicht aufgebracht. Zum Schutz wird die Hafteinlage 1 beidseitig durch Deckfolien 4 abgedeckt, die zur Kennung der beiden unterschiedlichen Einlageseiten auch verschiedene Färbung besitzen können. Enthält der Haftstoff zusätzlich einen Weichmacher, läßt sich ein Austrocknen der Hafteinlage auch über lange Zeit sicher verhindern.

Zur Herstellung der Hafteinlagen 1 wird Haftstoff 3 kontinuierlich auf eine endlos zugeführte Faservliesbahn aufgetragen und dann mit einer zweiten Faservliesbahn abgedeckt, worauf dieses Dreischichtband zur Vergleichmäßigung der Haftstoffschicht und zum Durchsetzen der Faservliese mit dem Haftstoff unter geringer Druckeinwirkung zusammengepreßt wird. Dieses Band wird nun auf der dem Kiefer zugeordneten Faservliesschicht nochmals mit Haftstoff bestrichen und dann beidseitig durch Deckfolien abgedeckt. Das so vorgefertigte Band gelangt anschließend in eine Prägestanzeinrichtung, in der entsprechende U-förmige Stanzlinge ausgestanzt werden, die bereits gebrauchsfertige Hafteinlagen 1 bilden. Durch das Stanzen kommt es zu einem randseitigen Zusammendrücken der Deckfolien 4, so daß eine geschlossene Schutzhülle entsteht. Nach einem abschließenden luftdichten Abpacken der Stanzlinge, beispielsweise durch ein Einschweißen in Packfolien, ist die Hafteinlage verkaufsbereit.

Zum Gebrauch wird die Hafteinlage 1 der Verpackung entnommen und nach einem Abziehen der unteren, prothesenseitigen Deckfolie 4 auf die Prothese aufgedrückt, wobei erforderlichenfalls die Einlage der Prothesenkontur folgend zuzuschneiden ist. Durch das Andrücken der Einlage an die Prothese tritt Haftstoff durch die untere Faservliesschicht 2 hindurch und gewährleistet die Haftverbindung zwischen Einlage 1 und Prothese. Wird nun die obere Deckfolie 4 abgezogen und die Prothese samt der Hafteinlage 1 eingesetzt, ergibt sich ein sicherer, haltbarer Prothesensitz, da der Haftstoff 3 durch Feuchtigkeitsaufnahme aufquillt und eine gummiartige, faserverfestigte Masse entsteht, die sowohl einen reinen Klebeeffekt als auch einen unterdruckbedingten Hafteffekt mit sich bringt.

## Ansprüche

1. Hafteinlage (1) für Zahnprothesen, insbesondere Unterkiefer-Zahnprothesen, bestehend aus einem haftstoffdurchsetzen Faservlies (2, 3), dadurch gekennzeichnet, daß das Faservlies (2) einen Haftstoff (3) aus einer pastösen Mischung von Alginat und/oder Carboxymethylcellulose, Polyvinylacetat und einem alkoholischen Lösungsmittel aufnimmt.

2. Hafteinlage nach Anspruch 1, dadurch gekennzeichnet, daß der Haftstoff (3) einen physiologisch einwandfreien Weichmacher, vorzugsweise ein Propylen-Glykol oder ein Glycerin-Triacetat enthält.

3. Hafteinlage nach Anspruch 1 oder 2, dadurch gekennzeichnet, daß Haftstoff (3) zwischen zwei Faservliesschichten (2) eingebracht und auf die dem Kiefer zugeordnete Faservliesschicht außen aufgetragen ist.

4. Hafteinlage nach Anspruch 3, dadurch gekennzeichnet, daß auf die äußere Haftstoffschicht (3) und vorzugsweise auch die äußere Faservliesschicht (2) Deckfolien (4) aufgelegt sind, welche beiden Deckfolien (4) gegebenenfalls unterschiedliche Farbe besitzen.

5. Verfahren zum Herstellen einer Hafteinlage nach einem der Ansprüche 1 bis 4, dadurch gekennzeichnet, daß eine Lösung auf feinzerteiltem Polyvinylacetat und vorzugsweise 85%-igem Äthylalkohol vorbereitet, in diese Lösung Alginat und/oder Carboxymethylcellulose eingebracht und die ganze Mischung bis zu einer pastösen Masse geknetet wird, mit welcher Masse dann Faservlies durchsetzt bzw. bestrichen wird.

Verfahren nach Anspruch 5, dadurch gekennzeichnet, daß eine Lösung aus etwa 57% Polyvinylacetat und 43% Äthylalkohol aufbereitet wird und etwa 50 Gewichtsteile dieser Lösung mit 35 Gewichtsteilen Alginat oder Carboxymethylcellulose bzw. 7 Gewichtsteilen Alginat und 28 Gewichtsteilen Carboxymethylcellulose vermischt werden.

7. Verfahren nach Anspruch 5 oder 6, dadurch gekennzeichnet, daß zu 100 Gewichtsteilen der fertigen Polyvinylacetatlösung zumindest 50 Gewichtsteile des Weichmachers mittels Vakuum-Rührer zugemischt werden.

8. Verfahren nach einem der Ansprüche 5 bis 7, dadurch gekennzeichnet, daß Haftstoff kontinuierlich auf eine Faservliesbahn aufgetragen, mit einer zweiten Faservliesbahn abgedeckt und leicht verpreßt wird, worauf die zweite Faservliesbahn mit Haftstoff bestrichen und das entstehende Band aus Faservlies- und Haftstoffschichten vorzugsweise beidseitig mit Deckfolien versehen wird, aus welchem Band anschließend der Prothesenform entsprechende Stanzlinge ausgestanzt und diese als gebrauchsbereite Hafteinlage stückweise luftdicht abgepackt werden.

9. Verfahren nach Anspruch 8, dadurch gekennzeichnet, daß Faservliesbahnen aus Zellulose mit einem Flächengewicht von ca. 45 g/m² bzw. 70 g/m² vorgesehen sind, auf deren erste Haftstoff in einer Menge von ca. 8 g/dm² auf deren zweite Haftstoff in einer Menge von ca. 5 g/dm² aufgetragen wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-B-1 566 252 (NEDWIG) * Spalte 1, Zeile 53 - Spalte 3, Zeile 3 * | 1 | A 61 C 13/23 |
| | --- | | |
| D,Y | DE-C-3 546 367 (ALTWIRTH) * Gesamtes Dokument * | 1 | |
| D,A | | 2,5 | |
| | --- | | |
| D,A | DE-B-2 413 380 (NEDWIG) * Ansprüche 1,3-6 * | 1,3,5,8 | |
| | --- | | |
| A | DE-U-1 992 630 (DUSSARDIER) * Seite 4, Zeilen 6-18, Figuren 1,2 * | 1,4 | |
| | --- | | |
| A | US-A-4 503 116 (LAPIDUS) * Spalte 3, Zeile 42 - Spalte 4, Zeile 6; Figuren 3,4 * | 1,5,8 | |
| | --- | | |
| A | US-A-2 664 631 (HOLLANDER et al.) * Spalte 6, Zeilen 29-36 * | 1,8 | |
| | ----- | | |

RECHERCHIERTE SACHGEBIETE (Int. Cl.4)

A 61 C 13/00
A 61 K 6/00

Der vorliegende Recherchenbericht wurde für alle Patentansprüche erstellt

| Recherchenort | Abschlußdatum der Recherche | Prüfer |
|---|---|---|
| BERLIN | 10-03-1989 | SIMON J J P |

EPO FORM 1503 03.82 (P0403)